(19) Europäisches Patentamt
European Patent Office
Office européen des brevets

(11) Numéro de publication: **0 050 890**
**A1**

(12) # DEMANDE DE BREVET EUROPEEN

(21) Numéro de dépôt: **81201140.1**

(22) Date de dépôt: **13.10.81**

(51) Int. Cl.³: **C 07 C 17/42**
C 07 C 19/05, C 09 K 15/30

(30) Priorité: **23.10.80 FR 8022749**

(43) Date de publication de la demande:
**05.05.82 Bulletin 82/18**

(84) Etats contractants désignés:
**AT BE CH DE FR GB IT LI NL SE**

(71) Demandeur: **SOLVAY & Cie (Société Anonyme)**
**Rue du Prince Albert, 33**
**B-1050 Bruxelles(BE)**

(72) Inventeur: **Ryckaert, André**
**Avenue Jacques Pastur, 132**
**B-1180 Bruxelles(BE)**

(72) Inventeur: **Servais, Michel**
**Chaussée de Malines, 315**
**B-1950 Kraainem(BE)**

(74) Mandataire: **Eischen, Roland**
**Solvay & Cie Département de la Propriété Industrielle**
**Rue de Ransbeek 310**
**B-1120 Bruxelles(BE)**

(54) **Compositions stabilisées de solvants chlorés.**

(57) L'invention concerne des compositions de solvants chlorés tels que le 1,1,1-trichloréthane stabilisées au moyen d'un composé hétérocyclique contenant des unités oxaziridines tel que la 2-tert-butyloxaziridine.

EP 0 050 890 A1

Croydon Printing Company Ltd.

# Compositions stabilisées de solvants chlorés

Cas S.80/10

SOLVAY & Cie (Société Anonyme)

La présente invention concerne des compositions stabilisées de solvants chlorés tels que le chlorure de méthylène, le trichloréthylène, le perchloréthylène et le 1,1,1-trichloréthane.

On sait que l'on peut obtenir des compositions stables de solvants chlorés tels que le chlorure de méthylène, le trichloréthylène, le perchloréthylène et le 1,1,1-trichloréthane, qui peuvent être utilisées notamment pour le dégraissage des métaux, le nettoyage à sec des textiles et dans les aérosols, en ajoutant au solvant chloré des produits chimiques appelés stabilisants et pouvant être choisis parmi une multitude de composés chimiques de natures variées. Suivant le solvant chloré considéré et l'application auquel il est destiné, on choisit des stabilisants différents et on les met en oeuvre en quantités différentes. La sélection des meilleures formules de stabilisation se fait encore selon des méthodes largement empiriques. On observe toutefois, actuellement, une tendance générale pour tous les solvants chlorés qui consiste à recourir à des formules de stabilisation très complexes comportant un grand nombre de stabilisants différents, dans lesquelles ceux-ci sont utilisés dans des proportions importantes.

La présente invention vise à procurer, pour la stabilisation des solvants chlorés, de nouveaux stabilisants particulièrement efficaces qui permettent la simplification des formules de stabilisation et la réduction des quantités de stabilisant à mettre en oeuvre.

L'invention concerne à cet effet des compositions de solvants chlorés stabilisées au moyen d'un composé hétérocyclique contenant un cycle oxaziridine.

Par composé hétérocyclique contenant un cycle oxaziridine, on entend désigner n'importe quel composé chimique contenant au moins un cycle de formule

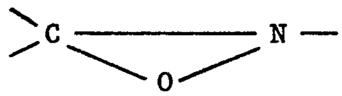

En général, on met en oeuvre un composé hétérocyclique de formule générale

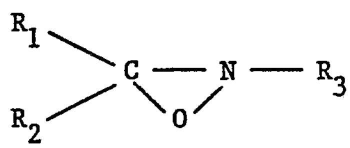

dans laquelle $R_1$, $R_2$ et $R_3$ sont des radicaux différents ou identiques, organiques ou inorganiques. De préférence, $R_1$, $R_2$ et $R_3$ représentent indépendamment les uns des autres un atome d'hydrogène, des radicaux aliphatiques ou cycloaliphatiques, saturés ou insaturés, éventuellement substitués par d'autres groupes ou atomes ou des radicaux aromatiques. Le plus souvent, $R_1$, $R_2$ et $R_3$ sont choisis parmi l'atome d'hydrogène, les radicaux alkyles, comprenant de 1 à 8 atomes de carbone et les radicaux aromatiques ne comprenant pas plus de 8 atomes de carbone. De préférence, $R_1$, $R_2$ et $R_3$ sont l'atome d'hydrogène ou des radicaux alkyles saturés comprenant de 1 à 5 atomes de carbone. Les composés tout particulièrement préférés sont ceux dont au moins un des radicaux $R_1$, $R_2$ ou $R_3$ est un radical alkyle ramifié. Enfin, parmi ceux-ci, les composés préférés sont ceux où au moins le radical $R_3$ est un radical alkyle ramifié. A titre d'exemples de composés convenant bien, on peut citer la 2-tert-butyloxaziridine, la 2-tert-amyloxaziridine, la 2-isopropyloxaziridine, la 3-méthyl-2-tert-butyloxaziridine, la 3-méthyl-2-tert-amyloxaziridine, la 3-méthyl-2-isopropyloxaziridine, la 3,3-diméthyl-2-tert-butyloxaziridine, la 3,3-diméthyl-2-tert-amyloxaziridine, la 3,3-diméthyl-2-iso.propyloxaziridine, la

3-méthyl-3-éthyl-2-tert-butyloxaziridine, la 3-méthyl-3-éthyl-2-tert-amyloxaziridine, la 3-méthyl-3-éthyl-2-isopropyloxaziridine, la 3-éthyl-2-tert-butyloxaziridine, la 3-éthyl-2-tert.amyloxaziridine, la 3-éthyl-2-isopropyloxaziridine, la 3-propyl-2-isopropyloxaziridine et la 3-isopropyl-2-isopropyloxaziridine. Parmi ceux-ci, la 2-tert-butyloxaziridine, la 2-tert-amyloxaziridine et la 2-isopropyloxaziridine conviennent le mieux.

Tout particulièrement préférée est la 2-tert-butyloxaziridine.

Les composés hétérocycliques, contenant le cycle oxaziridine, de l'invention peuvent être préparés par tout procédé connu à cet effet et notamment par les procédés décrits dans "Heterocyclic compounds with three and four membered rings" Part One -Chapter IV, Editor Arnold Weissberger 1964 Intersc. Publ. Wiley & Sons Inc. - New York -London - Sydney, pages 624 à 646.

La quantité de composé hétérocyclique à mettre en oeuvre varie suivant la nature du solvant chloré, suivant la nature des costabilisants utilisés et suivant l'application auquel le solvant chloré est destiné. Habituellement, cette quantité varie entre 0,0001 et 1 mole par litre de solvant chloré. Les quantités préférées se situent entre 0,001 et 0,5 mole/l et tout particulièrement préférées sont des quantités comprises entre 0,01 et 0,2 mole/l.

Les solvants chlorés qui peuvent être stabilisés selon l'invention peuvent être de natures très diverses. En général, ce sont des composés, aliphatiques ou aromatiques, contenant de 1 à 6 atomes de carbone et au moins un atome de chlore. Les solvants chlorés les plus usuels sont des dérivés chlorés du méthane, de l'éthane ou de l'éthylène tels que le chlorure de méthylène, le chloroforme, le 1,2-dichloréthane, le trichloréthylène, le perchloréthylène, et le 1,1,1-trichloréthane. L'invention se révèle particulièrement avantageuse dans le cas du 1,1,1-trichloréthane, qui a la réputation d'être le plus instable de ces solvants chlorés et qui est utilisé à grande échelle pour le dégraissage des métaux à chaud, à froid ou en phase vapeur.

Outre les composés hétérocycliques précités, les compositions stabilisées de solvants chlorés selon l'invention peuvent contenir

d'autres costabilisants convenant pour la stabilisation des solvants chlorés en question. Parmi les plus employés de ceux-ci, on peut citer les composés époxydés, les alcools, les nitroalcanes, les esters, les nitriles, les éthers internes, les cétones, les amines, les phénols et les composés oléfiniques.

Les composés époxydés utilisables comme costabilisants peuvent être saturés ou insaturés. Habituellement, ce sont des composés époxydés comprenant jusqu'à sept atomes de carbone du type saturé tels que l'époxypropane, l'époxybutane, les 2-méthyl-époxypropane, 2-méthylépoxybutanes et l'épichlorhydrine.

Les alcools utilisables comme costabilisants peuvent être saturés ou insaturés. Les alcools préférés contiennent de 1 à 7 atomes de carbone et peuvent être substitués ou non. Généralement, ce sont des alcools non substitués. Des exemples d'alcools saturés sont le méthanol, l'éthanol, le propanol, l'isopropanol, le butanol 1, le butanol 2, l'alcool butylique tertiaire, les pentanols tels que l'alcool amylique tertiaire, le méthylcellosolve, l'éthyl-cellosolve, le propylcellosolve, le butylcellosolve, la glycérine, l'éthylèneglycol, le propylèneglycol, la 2-hydroxy-2-méthyl-3-butanone, le 3,3-diméthoxy-2-méthylbutanol-2 et le 3-amino-2-méthyl-butanol-2. D'excellents résultats ont été obtenus avec les propanols, les butanols tertiaire et secondaire et le pentanol tertiaire. L'alcool saturé préféré est le butanol tertiaire. Les alcools insaturés habituellement utilisés sont des alcools non substitués contenant de 3 à 6 atomes de carbone. Parmi ceux-ci on préfère l'alcool allylique, le 2-méthyl-3-butène-2-ol. et le 2-méthyl-3-butyne-2-ol.

L'alcool insaturé particulièrement préféré est le 2-méthyl-3-butyne-2-ol.

Les nitroalcanes utilisables comme costabilisants contiennent habituellement de 1 à 4 atomes de carbone. De préférence, c'est le nitrométhane, le nitroéthane, les nitropropanes 1 ou 2 ou des mélanges de ces composés qui sont utilisés. Tout particulièrement préférés sont le nitrométhane et le nitroéthane.

Les esters utilisables comme costabilisants sont le plus souvent des mono- et des di-esters contenant de 2 à 8 atomes de

carbone saturés ou non, pouvant être substitués par des halogènes ou des groupes hydroxyles tels que les formiates d'éthyle, de propyle, d'isopropyle, de butyle, de sec-butyle, de tert-butyle, d'amyle, d'hexyle et de cyclohexyle, les acétates de méthyle, d'éthyle, de propyle, d'isopropyle, de butyle, d'amyle, d'éthynyle ou de propynyle, les propionates de méthyle, d'éthyle, de propyle, d'isopropyle, de butyle, d'amyle et d'éthynyle, les butyrates de méthyle, d'éthyle, de propyle, d'isopropyle et de 2-propynyle, les acrylates et méthacrylates de méthyle et d'éthyle, de propyle, d'isopropyle, de butyle, de sec-butyle et de tert-butyle, les crotonates et isocrotonates de méthyle et d'éthyle, les sorbiates d'éthyle et de méthyle, le diformiate d'éthyle et le diacétate d'éthyle. Les mono-esters non substitués contenant de 4 à 6 atomes de carbone conviennent bien. D'excellents résultats ont été obtenus avec l'acétate d'éthyle, l'acétate d'isobutyle, l'acétate de n-propyle, le méthacrylate de méthyle et l'acétate de n-butyle. Le méthacrylate de méthyle, l'acétate d'éthyle et l'acétate de n-propyle se sont révélés particulièrement adéquats.

Les nitriles utilisables comme costabilisants sont le plus souvent des composés aliphatiques ou aromatiques, saturés ou non saturés, éventuellement substitués, contenant de 2 à 7 atomes de carbone tels que l'acétonitrile, le propionitrile, l'acrylonitrile, le butyronitrile, le méthacrylonitrile, le malononitrile, le benzonitrile, le diméthylaminoacétonitrile, le méthylaminopropionitrile, le diméthylaminopropionitrile, le diéthylaminoacétonitrile, le méthyléthylaminoacétonitrile, le thiodipropionitrile, le tétracyanoéthylène, le tétracyanoquinodiméthane, le tétracyanodithiine, le 1,2-dicyano-1,2-bis(trifluorométhyl)éthylène, la cyanobenzaldéhyde, le nitrobenzonitrile, la cyanopyridine. Parmi ceux-ci l'acétonitrile, le propionitrile et l'acrylonitrile conviennent le mieux.

Les éthers internes particulièrement adéquats pour être utilisés comme costabilisants comprennent les éthers mono- ou polycycliques, pouvant contenir éventuellement d'autres hétéroatomes, contenant un ou des cycles à 5 ou 6 atomes de carbone saturés ou

non, substitués ou non tels que le 1,3-dioxanne, le 1,4-dioxanne, le 1,3,5-trioxanne, le 1,3-dioxolanne, le dioxène, le dioxadiène, le thioxanne, l'oxazole, le tétrahydrofuranne, l'oxyde de diphényle, etc.

Comme cétones, on peut utiliser les cétones contenant de 3 à 7 atomes de carbone substituées ou non, telles que par exemple l'acétone, la méthyléthylcétone, la diéthylcétone, la méthylpropylcétone, la méthylisopropylcétone, la 1(2 furyl)propanone, la 3-hydroxy-2,5-hexanedione, la 2-méthyl-2-hydroxy-4-pentanone,etc.

Comme amines, on utilise le plus souvent les composés comprenant de 3 à 10 atomes de carbone substitués ou non, telles que les amines aliphatiques secondaires et tertiaires ainsi que les amines aromatiques telles que l'aniline éventuellement substituée par des groupements alkyles en $C_1$ à $C_4$ ou des atomes de chlore. Les amines aliphatiques préférées sont la triéthylamine, la diisopropylamine, l'amylamine et l'héxylamine. Au titre d'amines on peut également utiliser des composés hétérocycliques comportant un atome d'azote dans le cycle. Parmi ceux-ci on utilise habituellement ceux contenant de 4 à 12 atomes de carbone et 1 à 2 atomes d'azote dans la molécule, tels que la pyridine, les picolines, la phénothiazine, la morpholine et la pyrrolidine ainsi que leurs dérivés substitués par des groupements alkyles sur l'atome d'azote. Enfin, les amines tout particulièrement préférées sont la N-méthylmorpholine, la diisopropylamine, la pyrrolidone et le N-méthylpyrrole.

Les composés phénoliques habituellement utilisés peuvent comporter un ou plusieurs groupes hydroxyles liés à un noyau benzénique éventuellement déjà substitué par des groupements alkyles. Les composés préférés sont le phénol et ses dérivés alkylés tels que le thymol, les crésols, le para-méthoxyphénol, les méthyl-di-tert.butylphénols et les diméthyl-tert.butylphénols. Tout particulièrement préférés sont le phénol, le thymol et le p.méthoxyphénol.

Enfin les composés oléfiniques utilisables comme costabilisants sont en général des composés comportant une ou plusieurs doubles

liaisons dans la molécule et comportant de 4 à 10 atomes de carbone. Parmi ceux-ci les composés préférés sont l'amylène tertiaire et le diisobutylène.

D'autres composés et notamment les alkoxyalcanes tels que les alkoxyéthanes, les nitrates tels que les nitrates d'alkyle contenant de 1 à 5 atomes de carbone, les quinones telles que l'hydroquinone et les anthraquinones, les hydrazines telles la diméthylhydrazine et des composés aromatiques tels que le toluène peuvent également être utilisés comme costabilisants.

Ces différents costabilisants utilisés sont généralement présents dans des quantités très variables comprises entre 0,001 et 50 g/l de solvant chloré.

Les compositions des solvants chlorés stabilisées selon l'invention conviennent particulièrement bien pour les applications requérant simultanément une bonne stabilisation du solvant chloré considéré et une quantité totale de stabilisants présents aussi faible que possible. Elles sont utilisées avantageusement pour dégraisser les métaux à chaud, à froid ou en phase vapeur et tout particulièrement pour le dégraissage des métaux à froid étant donné que les compositions des solvants chlorés stabilisées selon l'invention présentent la particularité de mouiller très efficacement les pièces métalliques.

Comme exemples de compositions de solvants chlorés stabilisées selon l'invention, on peut citer :

- du 1,1,1-trichloréthane, une oxaziridine telle que la 2-tert-butyloxaziridine, un nitroalkane tel que le nitrométhane ou le nitroéthane, un alcool aliphatique saturé tel que l'alcool butylique ou l'alcool amylique tertiaire et un alcool aliphatique insaturé tel que le 2-méthyl-3-butyne-2-ol.;
- du trichloréthylène, une oxaziridine telle que 2-tert-butyloxaziridine, une ou plusieurs amines, un ester tel que l'acétate d'éthyle et un ou plusieurs phénols;
- du perchloréthylène, une oxaziridine telle que la 2-tert.butyl ou 2-tert-amyloxaziridine, un ou plusieurs composés phénoliques, un ou plusieurs époxydes et une ou plusieurs amines;

- du chlorure de méthylène, une oxaziridine telle que la 2-propyl-oxaziridine ou la 2-tert-butyloxaziridine, un ou plusieurs époxydes, une ou plusieurs amines, un ester, du nitrométhane, un éther interne contenant un ou deux atomes d'oxygène et éventuellement un composé oléfinique.

Exemple 1 - Cet exemple est fourni à titre comparatif.

Essai 1

Dans un ballon de 500 $cm^3$ équipé d'un thermomètre et muni d'un ajutage de CLAISEN, destiné au blocage de réactions de décompositions exothermiques, surmonté d'un réfrigérant à reflux équipé d'un sécheur garni de grains de $CaCl_2$ anhydre, on introduit 100 $cm^3$ de 1,1,1-trichloréthane, stabilisé par 40 g/l d'alcool butylique tertiaire, 17,5 g/l de 2-méthyl-3-butyne-2-ol., 10 g/l de nitrométhane et 5,7 g/l d'époxybutane et 100 $cm^3$ de toluène. On ajoute à ce mélange 18 g de paillettes d'aluminium et 0,7 g de $AlCl_3$.

Ce mélange est soumis à l'ébullition à reflux durant 18 h. avec arrêt la nuit et aucune décomposition n'est observée.

Essai 2

On répète l'essai 1 mais en mettant en oeuvre le 1,1,1-trichloréthane, stabilisé par 20 g/l d'alcool butylique tertiaire, 8,75 g/l de 2-méthyl-3-butyne-2-ol., 5 g/l de nitrométhane et 5,7 g/l d'époxybutane.

Ce mélange est également soumis à l'ébullition à reflux et subit une décomposition exothermique après 12 heures.

Exemple 2

Essai 1

Dans les mêmes conditions que décrites pour l'exemple 1, essai 1 on met en oeuvre le 1,1,1-trichloréthane, stabilisé par 20 g/l d'alcool butylique tertiaire, 8,75 g/l de 2-méthyl-3-butyne-2-ol., 5 g/l de nitrométhane et 8,0 g/l de 2-tert-butyloxaziridine.

Après 18 heures d'ébullition à reflux, avec arrêt la nuit, aucune décomposition n'est observée.

Essai 2

On répète l'essai 1 mais on met en oeuvre une formule de stabilisation ne contenant que 5,6 g/l de 2-tert-butyloxaziridine et on constate que de nouveau qu'il n'y a pas de décomposition du 1,1,1-trichloréthane dans les conditions précitées.

On peut déduire de la comparaison des résultats des exemples 1 et 2 que l'emploi d'une oxaziridine dans une formule de stabilisation usuelle en lieu et place du composé époxydé permet de réduire au moins de moitié la quantité des autres stabilisants nécessaires pour l'obtention d'une bonne stabilité.

REVENDICATIONS

1 - Compositions stabilisées de solvants chlorés, caractérisées en ce qu'elles sont stabilisées au moyen d'un composé hétérocyclique contenant un cycle oxaziridine.

2 - Compositions selon la revendication 1, caractérisées en ce que le composé hétérocyclique répond à la formule générale

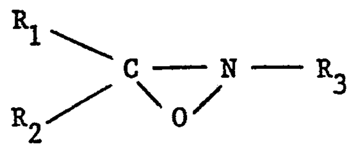

dans laquelle $R_1$, $R_2$ et $R_3$ sont des radicaux différents ou identiques, organiques ou inorganiques.

3 - Compositions selon la revendication 1 ou 2, caractérisées en ce que $R_1$, $R_2$ et $R_3$ sont choisis parmi l'atome d'hydrogène, les radicaux alkyles, comprenant de 1 à 8 atomes de carbone et les radicaux aromatiques ne comprenant pas plus de 8 atomes de carbone.

4 - Compositions selon l'une quelconque des revendications 1 ou 2, caractérisées en ce que au moins un des radicaux $R_1$, $R_2$ ou $R_3$ est un radical alkyle ramifié.

5 - Compositions selon la revendication 4, caractérisées en ce que au moins $R_3$ est un radical alkyle ramifié.

6 - Compositions selon l'une quelconque des revendications 1 à 5, caractérisées en ce que le composé hétérocyclique est la 2-tert-butyloxaziridine, la 2-tert-amyloxaziridine, ou la 2-isopropyloxaziridine.

7 - Compositions selon l'une quelconque des revendications 1 à 6, caractérisées en ce que le composé hétérocyclique est présent à raison de 0,001 mole à 0,5 mole par litre de solvant chloré.

8 - Compositions selon la revendication 7, caractérisées en ce que le composé hétérocyclique est présent à raison de 0,01 à 0,2 mole par litre de solvant chloré.

9 - Compositions selon l'une quelconque des revendications 1 à 8, caractérisées en ce que le solvant chloré est le 1,1,1-trichloréthane.

10 - Compositions selon la revendication 9, caractérisées en ce qu'elles sont stabilisées au moyen d'une oxaziridine, d'un alcool aliphatique saturé, un alcool aliphatique insaturé et un nitroalkane.

0050890

Office européen des brevets

# RAPPORT DE RECHERCHE EUROPEENNE

EP 81 20 1140

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
| --- | --- | --- | --- |
| A | BE - A - 638 938 (PITTSBURGH PLATE GLASS COMPANY)<br>* pages 6-8; revendications 6,23 * |  | C 07 C 17/42<br>C 07 C 19/05<br>C 09 K 15/30 |
| A | FR - A - 2 108 015 (SOLVAY & CIE.) |  |  |

DOMAINES TECHNIQUES RECHERCHES (Int. Cl. 3)

C 07 C 17/42
C 09 K 15/30

CATEGORIE DES DOCUMENTS CITES

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention
E: document de brevet antérieur, mais publié à la date de dépôt ou après cette date
D: cité dans la demande
L: cité pour d'autres raisons

&: membre de la même famille, document correspondant

X Le présent rapport de recherche a été établi pour toutes les revendications

| Lieu de la recherche | Date d'achèvement de la recherche | Examinateur |
| --- | --- | --- |
| La Haye | 19.01.1982 | VAN GEYT |